Europäisches Patentamt

(19) European Patent Office

Office européen des brevets

(11) Publication number: **0 047 398**
**B1**

(12) **EUROPEAN PATENT SPECIFICATION**

(45) Date of publication of patent specification: **19.12.84**

(51) Int. Cl.³: **A 61 M 5/24**

(21) Application number: **81106128.2**

(22) Date of filing: **05.08.81**

(54) Hypodermic cartridge syringe.

(30) Priority: **05.08.80 JP 106806/80**

(43) Date of publication of application:
**17.03.82 Bulletin 82/11**

(45) Publication of the grant of the patent:
**19.12.84 Bulletin 84/51**

(84) Designated Contracting States:
**AT BE CH DE FR GB IT LI LU NL SE**

(56) References cited:
**DE-C- 217 162**
**FR-A-2 223 051**
**GB-A-1 103 917**
**US-A-2 514 575**
**US-A-3 110 309**

(73) Proprietor: **COLPO COMPANY LIMITED**
**8th Floor Pine Central Bldg. No.11-5, 2-chome**
**Kyobashi, Chuo-ku, Tokyo (JP)**

(72) Inventor: **Takasugi, Mitsuo**
**No. 74, Kuritaya Kanagawa-ku**
**Yokohama Kanagawa (JP)**
Inventor: **Okuyama, Yoshio**
**No. 13-7, 3-chome, Shimouma**
**Setagaya-ku Tokyo (JP)**

(74) Representative: **Klingseisen, Franz, Dipl.-Ing.**
**et al**
**Dr. F. Zumstein sen. Dr. E. Assmann Dr. R.**
**Koenigsberger Dr. F. Zumstein jun. Dipl.-Ing. F.**
**Klingseisen Bräuhausstrasse 4**
**D-8000 München 2 (DE)**

Courier Press, Leamington Spa, England.

**Description**

The invention refers to a hypodermic cartridge syringe according to the preamble of claim 1.

Such a syringe is known from US—A—2 514 575. The guiding element for the piston is embodied as a plug inserted into the elongated syringe tube and having a bore for guiding the piston in the form of a rod shiftable in this bore, the rod having a convex end engaging the bottom of the cartridge. After use of the syringe it is difficult to remove the cartridge from the elongated syringe tube.

Further it is known from FR—A—2 223 051 to secure the needle carrier by means of a rib and grooves on a syringe tube in such a way that the needle is advanced step by step to pierce a seal and to arrive at a dispersing position.

It is the object of the invention to improve a syringe according to the preamble of claim 1 in such a way that the handling of the syringe especially in emergency cases is easier as to inserting and removing the cartridge.

That is achieved according to the invention by the features in the characterizing part of claim 1. Such a structure facilitates the replacing of the cartridge in the syringe tube. Thus a syringe is provided, which may be reused only by exchanging the cartridge without disinfection each time.

An example of the invention is shown in detail in the drawings.

Fig. 1 is a vertical cross sectional view of a preferred embodiment of the invention,

Fig. 2 is a view showing disassembly of the above mentioned embodiment, and

Fig. 3 is an enlarged vertical cross sectional view showing an operation of the embodiment.

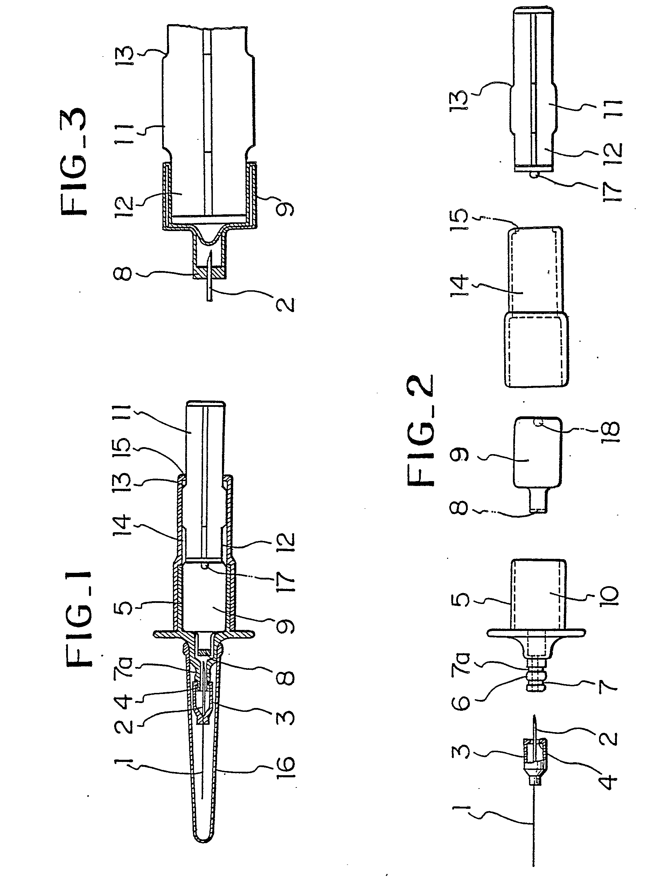

The syringe is composed of a needle carrier, a syringe tube, a cylinder and a piston. The needle carrier 3 has a hollow needle 2 of a desired length extending in opposite direction to the needle 1, and is provided with a ring projection 4 within a fitting portion to be engaged with a needle connecting portion 6 on the neck portion of the syringe tube 5. The injecting tube 5 is provided with more than one of grooves 7 and 7a on the connecting portion 6, and is formed with a hollow body portion 10 to be charged with an ampoule-like cartridge 9 with a sealing cover 8 holding the liquid as shown in fig. 2. With respect to the needle, the projection 4 engages the groove 7 into restraint, and accordingly the hollow needle 2 is positioned to have its end portion slightly spaced from the sealing cover 8. The piston has an enlarged diameter portion 11 and an engaging convex 17 on a head 12 thereof, the head 12 being narrower in diameter than an inner diameter of the hollow body portion 10 over more than 1/2 of the depth of the body portion 10. A step 13 is provided on a sliding surface following the head 12. The position of the step 13 is restrained at a reduced diameter end opening 15 which defines a stop on a cylinder 14. The cylinder 14 includes a large diameter portion and a small diameter portion. The large diameter portion is adapted to be coaxially positioned about the body of the syringe tube 5, and the piston is inserted into the small diameter portion from the large diameter portion. The small diameter portion has the stop at a circumferential edge of an opening thereof, so that the piston contacts, when moving in opposition, to the step 13 and does not get out from the cylinder 14.

The syringe tube 5 is air-tightly mounted with a needle protecting cap 16 on a neck thereof. The cartridge 9 has an engaging recess 18 in correspondence to an engaging protuberance 17 of the piston. The positioning relation of the protuberance 17 and the recess 18 may be reversed, that is, it is allowable to make a protuberance on the bottom of the cartridge 9 and make a recess on the head of the piston.

The syringe of this invention is constructed as mentioned above, and is set up by positioning the cartridge 9 into the hollow body portion 10 of the syringe tube 5 having the needle, and inserting and pushing the piston into the cylinder 14 from its large diameter portion until the step 13 contacts the stop 15. Then, the large diameter portion of the cylinder 14 is positioned coaxially of and radially outwardly of the syringe tube 5, and the piston pushed slightly so that the protuberance 17 is engaged with the recess 18 and thus the assembly of the syringe is completed (Fig. 1). For performing the injection, the cap 16 is removed and the pressure is effected to the needle 1 toward the tube 5, so that the fitting portion 4 is urged into the other groove 7a from the groove 7, whereby the hollow needle 2 pierces the sealing cover 8 of the cartridge 9 and reaches the liquid agent therein. Then, when pushing the piston on its end portion, the liquid agent in the cartridge 9 goes to the needle 1 through the hollow needle 2. As the piston head 12 is pushing the bottom of the cartridge 9 while the protuberance 17 is engaged with the recess 18, the cartridge film overlaps by turning over itself (doubles over) and makes a double film, and the liquid agent in the cartridge 9 exhausts all the amount up to its throat. This amount is a predetermined amount. If the piston is moved back just after the injection and since the engagement between the protuberance 17 and the recess 18 is maintained, the bottom of the cartridge 9 is pulled by the piston. Therefore, it is permitted to move back the piston and it is possible to confirm whether the injection is properly made in the blood vessel.

The present invention makes, by one action of fitting the needle, a connection of the liquid agent in the cartridge and the hollow needle and further the injecting needle. Therefore, the needle goes into the ampoule, and the liquid never touches other members. This is very hygienic and being without disinfection and possible to use it only by charging the cartridge, this is very suitable for emergencies outdoors, and further the liquid does not leak at all. The invention is much suited to emergencies, since it may omit time and steps for conventionally breaking an ampoule at its neck, absorbing the liquid into the syringe, expelling the air from the tube and injecting the liquid. The invention does not need to pay attention to the precision of the air-tight sliding between the cylinder and the piston or to the air tight packing of the piston head, and no precision is required for any parts. Also in the instant syringe, transparency is preferable, but opacity for heat and light insulation in view of the weather resistability of the liquid is not impreferable, and it is sufficient to make a mark for the liquid volume at the end of the piston. The cartridge is pushed by the piston and the cartridge film is turned over between the syringe tube and the piston head. This condition corresponds to the slide contacting surface between the cylinder and the piston, so that the liquid is all exhausted. Thus, the invention is very useful to calamities such as earthquakes, mountain-climbing, travelling, fire accidents and others. This invention is an excellent syringe in safety, stability and function.

## Claims

1. Syringe comprising a needle carrier (3), a syringe tube (5) having a neck portion (6) and a hollow body portion (10) open at the end thereof opposite said neck portion, the needle carrier (3) being detachably secured to said neck portion of the syringe tube (5), said hollow body portion (10) of the syringe tube being adapted to receive a cartridge (9) on the interior of said body portion, and a piston having a piston head (12) engageable with the bottom of the cartridge (9), the piston being connected with the syringe tube (5) by a guiding element (14), the piston head (12) having a smaller diameter than the inner diameter of the syringe tube (5) for doubling over the wall of said cartridge due to the pressure exerted by the piston, characterized in that the guiding element for the piston has the form of a cylinder (14) having a first cylinder body portion receiving the hollow body portion (10) of the syringe tube (5), said cylinder (14) having a second cylinder body portion contiguous and communicating with said first body portion for guiding the piston and that the piston is provided with an enlarged diameter portion (11) intermediate the length thereof and the cylinder (14) is provided with an open end (15) of smaller diameter than the enlarged diameter portion (11) of the piston for defining a stop, the piston head normally being positioned substantially within the second body portion of the cylinder (14), but movable into said syringe tube (5).

2. Syringe according to claim 1, characterized in that the cartridge (9) and said piston head (12) define two cooperating members, one of said members having a protuberance (17) thereon in facing relation to the other of said members, said other member having a recess (18) therein which is engaged by said protuberance of said one member, whereby to engage said piston head with said cartridge.

3. Syringe according to claim 1 or 2, characterized in that the neck portion (6) of the syringe tube (5) includes a first groove (7) and a second groove (7a) in spaced relation from each other, a fitting portion (4) in supporting relation to the needle carrier (3), said fitting portion (4) being initially engageable with said first groove (7) to maintain said needle carrier in spaced relation to said second groove (7a) when the syringe is being used for performing an injection.

## Revendications

1. Seringue comprenant un porte-aiguille (3), un tube de seringue (5) présentant une partie de collet (6) et une partie de corps creux (10) ouverte à son extrémité opposée à ladite partie de collet, le porte-aiguille (3) pouvant être fixé de façon amovible à ladite partie de collet du tube de seringue (5), ladite partie de corps creux (10) du tube de seringue étant agencée pour recevoir une cartouche (9) à l'intérieur de ladite partie de corps, et un piston comportant une tête de piston (12) pouvant venir en contact avec le fond de la cartouche (9), le piston étant relié au tube de seringue (5) par un élément de guidage (14), la tête de piston (12) ayant un diamètre plus faible que le diamètre interne du tube de seringue (5) en vue du repli sur elle-même de la paroi de ladite cartouche en raison de la pression exercée par le piston, caractérisée en ce que l'élément de guidage du piston a la forme d'un cylindre (14) comprenant une première partie de corps de cylindre recevant la partie de corps creux (10) du tube de seringue (5), ledit cylindre (14) comprenant une seconde partie de corps cylindrique contigué et communiquant avec ladite première partie de corps pour guider le piston, et en ce que le piston est muni d'une partie de diamètre plus important (11) dans la partie centrale de sa longueur, et le cylindre (14) est muni d'une extrémité ouverte (15) de diamètre inférieur à la partie de diamètre plus important (11) dudit piston pour définir une butée, la tête du piston étant normalement disposée sensiblement à l'intérieur de la seconde partie de corps du cylindre (14), mais pouvant se déplacer dans ledit tube de seringue (5).

2. Seringue selon la revendication 1, caractérisée en ce que la cartouche (9) et ladite tête de piston (12) définissent deux organes coopérants, l'un desdits organes comprenant une saillie (17) formée sur lui et faisant face à l'autre desdits organes, ledit autre organe comprenant un évidement (18) qui peut venir en engagement avec ladite saillie dudit autre organe, de manière que ladite tête de piston vienne en contact avec ladite cartouche.

3. Seringue selon la revendication 1 ou 2, caractérisée en ce que la partie de collet (6) du tube de seringue (5) comprend une première gorge (7) et une seconde gorge (7a) espacées l'une de l'autre, une partie de fixation (4) qui supporte le porte-aiguille (3), ladite partie de fixation (4) pouvant être initialement engagée dans ladite première gorge (7) pour maintenir ledit porte-aiguille espacé de ladite seconde gorge (7a) servant quand la seringue est utilisée pour effectuer une injection.

**Patentansprüche**

1. Spritze mit einem Nadelträger (3), einer Spritzenröhre (5) mit einem Halsteil (6) und einem Hohlkörperteil (10), der an einem Ende dem Halsteil gegenüber offen ist, wobei der Nadelträger (3) abnehmbar am Halsteil der Spritzenröhre (5) befestigt ist, der Hohlkörperteil (10) der Spritzenröhre so ausgebildet ist, daß er eine Patrone (9) im Inneren des Körperteils aufnehmen kann, und mit einem Kolben mit Kolbenkopf (12), der mit dem Boden der Patrone in Eingriff bringbar ist, wobei der Kolben mit der Spritzenröhre (5) über ein Führungselement (14) verbunden ist und der Kolbenkopf (12) einen Durchmesser hat, der kleiner als der Innendurchmesser der Spritzenröhre (5) ist, um die Wand der Patrone aufgrund des durch den Kolben ausgeübten Druckes doppelt übereinander zu legen, dadurch gekennzeichnet, daß das Führungselement für den Kolben die Form eines Zylinders (14) mit einem ersten Zylinderkörperteil hat, der den Hohlkörperteil (10) der Spritzenröhre (5) aufnimmt, der Zylinder (14) einen zweiten Zylinderkörperteil aufweist, der sich an den ersten Körperteil direkt anschließt und damit in Verbindung steht, um den Kolben zu führen, und daß der Kolben mit einem Teil (11) mit vergrößertem Durchmesser an einer Stelle in der Mitte seiner Länge versehen ist und der Zylinder (14) mit einem offenen Ende (15) mit kleinerem Durchmesser als dem des Teils (11) mit vergrößertem Durchmesser des Kolbens versehen ist, um einen Anschlag zu bilden, wobei der Kolbenkopf sich normalerweise im wesentlichen innerhalb des zweiten Körperteils des Zylinders (14) befindet, jedoch in die Spritzenröhre (5) hinein bewegbar ist.

2. Spritze nach Anspruch 1, dadurch gekennzeichnet, daß die Patrone (9) und der Kolbenkopf (12) zwei zusammenarbeitende Elemente bilden, wobei eines der Elemente einen Vorsprung (17) aufweist, der dem anderen Element zugewandt ist, und das andere Element eine Aussparung (18) aufweist, in die der Vorsprung des einen Elementes in Eingriff gebracht wird, wodurch der Kolbenkopf mit der Patrone in Eingriff kommt.

3. Spritze nach Anspruch 1 oder 2, dadurch gekennzeichnet, daß der Halsteil (6) der Spritzenröhre (5) eine erste Nut (7) und eine zweite Nut (7a) im Abstand voneinander aufweist, und ein Paßteil (4) in Haltebeziehung zum Nadelträger (3) vorgesehen ist, wobei der Paßteil (4) anfangs mit der ersten Nut (7) in Eingriff bringbar ist, um den Nadelträger im Abstand von der zweiten Nut (7a) zu halten, wenn die Spritze zur Ausführung einer Injektion benutzt werden soll.

# FIG_1

# FIG_3

# FIG_2